# EUROPÄISCHE PATENTSCHRIFT

(11) **EP 3 534 812 B1**
(45) Veröffentlichungstag und Bekanntmachung des Hinweises auf die Patenterteilung: **18.08.2021**
(21) Anmeldenummer: 17791365.4
(22) Anmeldetag: 26.10.2017
(51) Int. Cl.: A61B 17/70

(54) **VORRICHTUNG ZUM DURCHFÜHREN EINER DISTRAKTION ODER EINER KOMPRESSION VON WIRBELKÖRPERN BEI EINER WIRBELSÄULENOPERATION**
DEVICE FOR CARRYING OUT A DISTRACTION OR A COMPRESSION OF VERTEBRAL BODIES DURING SPINAL SURGERY
DISPOSITIF POUR LA RÉALISATION D'UNE DISTRACTION OU D'UNE COMPRESSION DE CORPS VERTÉBRAUX LORS D'UNE CHIRURGIE RACHIDIENNE

(30) Priorität: 04.11.2016 DE 102016121054
(43) Veröffentlichungstag der Anmeldung: 11.09.2019
(73) Patentinhaber: Silony Medical International AG, 8500 Frauenfeld (CH)
(72) Erfinder: OHNMACHT, Timo, 78736 Trichtingen (DE); RIESINGER, Ralf, 78234 Engen (DE)
(74) Vertreter: DREISS Patentanwälte PartG mbB
(86) Internationale Anmeldenummer: PCT/EP2017/077426
(87) Internationale Veröffentlichungsnummer: WO 2018/083000

(56) Entgegenhaltungen:
- US-A1- 2010 331 849
- US-A1- 2012 191 143
- US-A1- 2013 184 763
- US-A1- 2013 289 633
- US-A1- 2014 257 312

## Beschreibung

Die Erfindung betrifft eine Vorrichtung zum Durchführen einer Distraktion und/oder einer Kompression von Wirbelkörpern bei einer, insbesondere minimalinvasiven, Wirbelsäulenoperation, insbesondere mit einem ersten und einem zweiten, insbesondere zumindest abschnittsweise hülsenförmigen, Verlängerungsteil mit einer jeweiligen Längsrichtung, welches jeweils an einem Knochenanker zwar lösbar jedoch starr und drehfest festlegbar ist, wobei die Knochenanker jeweils in einen der zu behandelnden Wirbelkörper einzubringen oder bereits eingebracht sind, wobei eine Spreiz- oder Klemmeinrichtung mit den Oberbegriffsmerkmalen des Anspruchs 1 vorgesehen ist. Die vorgestellte Vorrichtung betrifft ein chirurgisch mehrfach verwendbares Instrument. Zur Wiederaufbereitung im Rahmen der Wiederverwendung gehören Schritte der Vorreinigung, der spülenden Hauptreinigung, der Sterilisation und der Trocknung. Im Rahmen der Erfindung wird nicht näher auf die einzelnen Arbeitsschritte eingegangen, vielmehr wird der Begriff Wiederaufbereitung verwendet.

In der modernen Wirbelsäulenchirurgie, insbesondere der minimal invasiven Wirbelsäulenchirurgie, wo mit hülsenförmigen Verlängerungsteilen der Knochenanker, mit sogenannten Extendern, gearbeitet wird, stellt sich häufig die Aufgabe, dass in der Regel benachbarte Wirbelkörper voneinander weg (Distraktion) oder aufeinander zu (Kompression) bewegt werden müssen. Die neue Position kann dann über Osteosynthesevorrichtungen mit Korrekturstäben fixiert werden. Hierfür gibt es im Stand der Technik teils aufwändig konstruierte Instrumente, welche die beiden Verlängerungsteile, die mit den in die Wirbelkörper eingebrachten Knochenankern starr verbunden sind, verstellbar zueinander verbinden. Die vorbekannten Vorrichtungen sind aufwändig herstellbar und komplex bedienbar. Eine Vorrichtung mit Merkmalen des Oberbegriffs des Anspruchs 1 ist bekannt aus US 2014/0257312 A1.

Eine weitere gattungsgemäße Vorrichtung ist aus US 2013/289633 A1 bekannt.

Der vorliegenden Erfindung liegt daher die Aufgabe zugrunde, eine Vorrichtung der eingangs genannten Art zu schaffen, mit welcher während der Distraktion oder Kompression zwar der Abstand der Wirbelkörper zueinander verändert werden kann, ihre Orientierung oder Ausrichtung relativ zueinander hingegen nicht oder zumindest nur geringfügig verändert wird, und die durch einen Chirurgen einfach bedienbar ist.

Diese Aufgabe wird durch eine Vorrichtung mit den Merkmalen des Anspruchs 1 gelöst. Eine derartige Vorrichtung zeichnet sich dadurch aus, dass die Spreiz- oder Klemmeinrichtung ein Grundteil mit einer Aufnahme zum lösbaren Festlegen der Spreiz- oder Klemmeinrichtung an dem ersten Verlängerungsteil aufweist und dass die Spreiz- oder Klemmeinrichtung ein Spreiz- oder Klemmteil mit einem Anlageabschnitt aufweist, der zur spreizenden und/oder klemmenden Anlage an dem zweiten Verlängerungsteil ausgelegt ist, und dass die Spreiz- oder Klemmeinrichtung eine Führung aufweist, entlang derer das Spreiz- oder Klemmteil gegenüber dem Grundteil verlagerbar ist, wobei die Spreiz- oder Klemmeinrichtung ein Schraubgetriebe mit einer Antriebswelle aufweist, das zur relativen Verlagerung des Spreiz- oder Klemmteils gegenüber dem Grundteil ausgelegt ist. Weiter ist vorgesehen, dass die Antriebswelle an ihrem dem Grundteil abgewandten freien Ende einen ersten Drehmomentantriebsabschnitt aufweist und dass ein Winkelgetriebe vorgesehen ist, das zum Antreiben der Antriebswelle an einem dem freien Ende abgewandten zweiten Ende ausgelegt ist. Der erste Drehmomentantriebsabschnitt ist dabei vorzugsweise koaxial zur Mittellängsachse der Antriebswelle angeordnet und kann als innenliegender Drehmomentantriebsabschnitt, insbesondere als Innensechskant, oder als außenliegender Drehmomentantriebsabschnitt, insbesondere als Drehgriff, ausgebildet sein. Somit kann die Antriebswelle einerseits mittels des ersten Drehmomentantriebsabschnitt koaxial zu ihrer Mittellängsachse rotierend angetrieben werden, wobei durch das Vorsehen des Winkelgetriebes eine weitere Möglichkeit des Antriebs geschaffen werden kann, mittels derer ein Antreiben der Antriebswelle in einem zusätzlichen Winkel gegenüber der Mittellängsachse der Antriebswelle möglich ist.

Vorteilhafterweise ist die Führung stabförmig ausgebildet und als von der Antriebswelle verschiedenes Teil ausgebildet. Weiterhin ist es vorteilhaft, wenn die Aufnahme als Ringklammer ausgebildet ist und derart ausgelegt ist, dass ein erstes hülsenförmiges Verlängerungsteil in seiner Längsrichtung klemmend darin aufgenommen werden kann. An der Aufnahme ist vorteilhafterweise eine Klemmschraube mit einer Flügelmutter angeordnet, wobei durch Anziehen der Flügelmutter eine Klemmkraft von der Aufnahme auf ein in der Aufnahme angeordnetes hülsenartiges Verlängerungsteil ausgeübt werden kann

Eine vorteilhafte Weiterbildung der Vorrichtung sieht vor, dass die Antriebswelle drehbar mit dem Grundteil verbunden ist, wobei die Antriebswelle eine Gewindespindel des Schraubgetriebes aufweist und wobei das Spreiz- oder Klemmteil ein Mutterngewinde des Schraubgetriebes aufweist. Als besonders vorteilhaft hat es sich dabei erwiesen, wenn die Gewindespindel und das Mutterngewinde derart miteinander korrespondieren, dass eine Rotation der Antriebswelle um ihre Mittellängsachse in eine axiale Verlagerung des Spreiz- oder Klemmteils gegenüber dem Grundteil entlang der Führung umgesetzt wird.

Weiterhin ist es vorteilhaft, wenn ein zweiter Drehmomentantriebsabschnitt vorgesehen ist, der am Grundteil angeordnet ist. Als besonders vorteilhaft hat es sich dabei erwiesen, wenn der zweite Drehmomentantriebsabschnitt insbesondere als Außentorx ausgebildet ist. Vorteilhafterweise ist darüber hinaus ein dritter Drehmomentantriebsabschnitt vorgesehen, der koaxial zum zweiten Drehmomentantriebsabschnitt angeordnet ist. Der zweite und/oder der dritte Drehmomentantriebsabschnitt sind dabei vorteilhafterweise zum Antreiben des Winkelgetriebes ausgelegt. Somit kann die Antriebswelle mittelbar durch Antreiben des ersten und/oder zweiten Drehmomentantriebsabschnitts in Rotation versetzt werden.

Für eine besonders einfache Bedienung der Spreiz- oder Klemmeinrichtung hat es sich als vorteilhaft erwiesen, wenn der zweite Drehmomentantriebsabschnitt orthogonal zum ersten Drehmomentantriebabschnitt angeordnet ist. Somit hat es sich als vorteilhaft erwiesen, wenn die Bauteile des Winkelgetriebes orthogonal zueinander angeordnet sind. Da sich der erster Drehmomentantriebsabschnitt vorteilhafterweise in Richtung der Längsachse der Antriebswelle erstreckt, ist es folglich bevorzugt, wenn sich der zweite Drehmomentantriebsabschnitt orthogonal zur Antriebswelle erstreckt.

Gemäß einer weiteren, besonders vorteilhaften Weiterbildung der Vorrichtung ist vorgesehen, dass das Winkelgetriebe als Kegelgetriebe oder Kronenradgetriebe ausgebildet ist. Dabei ist es denkbar, dass das Winkelgetriebe als Zahnradgetriebe ausgebildet ist. Alternativ dazu ist es jedoch auch denkbar, dass das Winkelgetriebe als Reibgetriebe ausgebildet ist.

Gemäß einer weiteren vorteilhaften Ausgestaltung der Vorrichtung ist vorgesehen, dass die Führung drehfest mit dem Grundteil verbunden ist. Besonders bevorzugt ist es dabei, wenn die Führung einstückig mit dem Grundteil verbunden ist.

Vorteilhafterweise ist die Antriebswelle parallel zur Führung angeordnet, so dass eine Rotation der Antriebswelle in eine axiale Bewegung des Spreiz- oder Klemmteils entlang der Führung umgesetzt werden kann.

Weiterhin ist es vorteilhaft, wenn im Grundteil eine erste Aufnahmebohrung für die Antriebswelle vorgesehen ist und wenn im Grundteil eine zweite Aufnahmebohrung für die Führung vorgesehen ist. Dabei ist es vorteilhaft, wenn das Mutterngewinde des Schraubgetriebes, das mit dem Gewinde der Gewindespindel korrespondiert, in der ersten Aufnahmebohrung angeordnet ist.

Um ein Wiederaufbereitung der Vorrichtung zu erleichtern hat es sich dabei als besonders vorteilhaft erwiesen, wenn die Führung einen ersten Führungsabschnitt mit einem ersten Führungsdurchmesser aufweist und wenn die Führung an ihrem freien Ende einen zweiten Führungsabschnitt mit einem zweiten Führungsdurchmesser aufweist, der kleiner ist als der erste Führungsdurchmesser. Um eine möglichst gute Führung des Spreiz- oder Klemmteils ermöglichen zu können, hat es sich als vorteilhaft erwiesen, wenn der erste Führungsdurchmesser nur geringfügig kleiner ausgebildet ist als ein Bohrungsdurchmesser der zweiten Aufnahmebohrung.

Um eine Wiederaufbereitung der Vorrichtung zusätzlich zu erleichtern hat es sich weiterhin als vorteilhaft erwiesen, wenn die Führung im Bereich des ersten Führungsabschnitts wenigstens eine, vorzugsweise drei Abflachungen aufweist, die orthogonal zueinander angeordnet sind. Des Weiteren erleichtern die Abflachungen die Lesbarkeit einer dort aufgebrachten Beschriftung. Besonders bevorzugt ist es dabei, wenn der erste Führungsabschnitt im Bereich der Abflachungen dennoch im Querschnitt kreisringabschnittsförmige Gleitabschnitte aufweist, die am Umfang der Führung zwischen den Abflachungen angeordnet sind. Somit kann einerseits eine möglichst gute Führung bzw. ein gutes Gleiten der Führung in der zweiten Aufnahmebohrung des Spreiz- oder Klemmteils gewährleistet werden, wobei andererseits Verschmutzungen durch bspw. menschliches Knochen- oder Gewebematerial einfach ausgespült werden können.

Um auch eine Wiederaufbereitung der Vorrichtung im Bereich der Antriebswelle erleichtern zu können, insbesondere um eine Wiederaufbereitung der Gewindespindel erleichtern zu können, hat es sich als vorteilhaft erwiesen, wenn die Antriebswelle einen ersten Antriebsabschnitt aufweist, in dem die Gewindespindel angeordnet ist, die einen Gewindespindeldurchmesser aufweist, und wobei die Antriebswelle im Bereich des freien Endes einen zweiten Antriebsabschnitt mit einem Antriebswellendurchmesser aufweist, der kleiner ist als der Gewindespindeldurchmesser. Vorteilhafterweise weisen der zweite Führungsabschnitt und der zweite Antriebsabschnitt dabei eine Länge auf, die derart ausgelegt ist, dass das Spreiz- oder Klemmteil vollständig vom ersten Führungsabschnitt bzw. vom ersten Antriebsabschnitt heruntergeschoben werden kann, so dass Verschmutzungen aus der ersten und zweiten Aufnahmebohrung entfernt werden können, wenn das Spreiz- oder Klemmteil auf den zweiten Führungsabschnitt bzw. den zweiten Antriebsabschnitt heruntergeschoben sind.

Besonders vorteilhaft ist es dabei, wenn der erste Führungsabschnitt und der erste Antriebsabschnitt in etwa dieselbe Länge aufweisen und wenn der zweite Führungsabschnitt und der zweite Antriebsabschnitt in etwa dieselbe Länge aufweisen.

Zur drehbaren Verbindung der Antriebswelle mit dem Grundteil kann vorgesehen sein, dass im Grundteil ein Lagerabschnitt vorgesehen ist, der zur drehbaren Lagerung der Antriebswelle ausgelegt ist.

Um ein zweites hülsenförmiges Verlängerungsteil manipulieren zu können, hat es sich als vorteilhaft erwiesen, wenn der Anlageabschnitt einen Klemmabschnitt aufweist, der dem Grundteil zugewandt ist, und wenn der Anlageabschnitt einen Spreizabschnitt aufweist, der dem Grundteil abgewandt ist. Besonders bevorzugt ist es dabei, wenn der Klemmabschnitt und der Spreizabschnitt zur insbesondere vollflächigen Anlage an einem hülsenförmigen Verlängerungsteil ausgelegt sind. Da die hülsenförmigen Verlängerungsteile zumindest abschnittsweise kreiszylindrisch ausgebildet sein können, ist es vorteilhaft, wenn der Anlageabschnitt bei Blick in Längsrichtung der Verlängerungsteile abschnittsweise doppelkonkav ausgebildet ist. Dabei ist die Oberfläche des in Längsrichtung gesehen konkav ausgebildeten Spreiz- oder Klemmabschnitts vorzugsweise in sich nochmals konvex gewölbt, so dass ein jeweiliges, am Spreiz- oder Klemmabschnitt zur Anlage kommendes hülsenförmiges Verlängerungsteil, das insbesondere zylindrisch ausgebildet ist, nicht plan am Spreiz- oder Klemmabschnitt zur Anlage kommt. Vielmehr erfolgt beim Verschränken/Neigen der hülsenförmigen Verlängerungsteile eine Art Abgleiten auf der in sich konvex gewölbten Oberfläche des konkaven Spreiz- oder Klemmabschnitts, so dass eine Art Sattelgelenk bereitgestellt werden kann.

Weitere Einzelheiten und vorteilhafte Weiterbildungen sind der nachfolgenden Beschreibung zu entnehmen, anhand derer eine Ausführungsform der Erfindung näher beschrieben und erläutert ist.

Es zeigen:
Figur 1 eine Schrägansicht einer erfindungsgemäßen Vorrichtung;
Figur 2 eine Schrägansicht einer Spreiz- oder Klemmeinrichtung der erfindungsgemäßen Vorrichtung gemäß Figur 1;
Figur 3 eine Schrägansicht der Spreiz- oder Klemmeinrichtung gemäß Figur 2 in einer Lage zur Wiederaufbereitung;
Figur 4 eine teilweise Explosionsdarstellung der Spreiz- oder Klemmeinrichtung gemäß der Figuren 2 und 3;
Figur 5 eine erste Detailansicht der Spreiz- oder Klemmeinrichtung gemäß der Figuren 2 bis 4; und
Figur 6 eine zweite Detailansicht der Spreiz- oder Klemmeinrichtung gemäß der Figuren 2 bis 4.

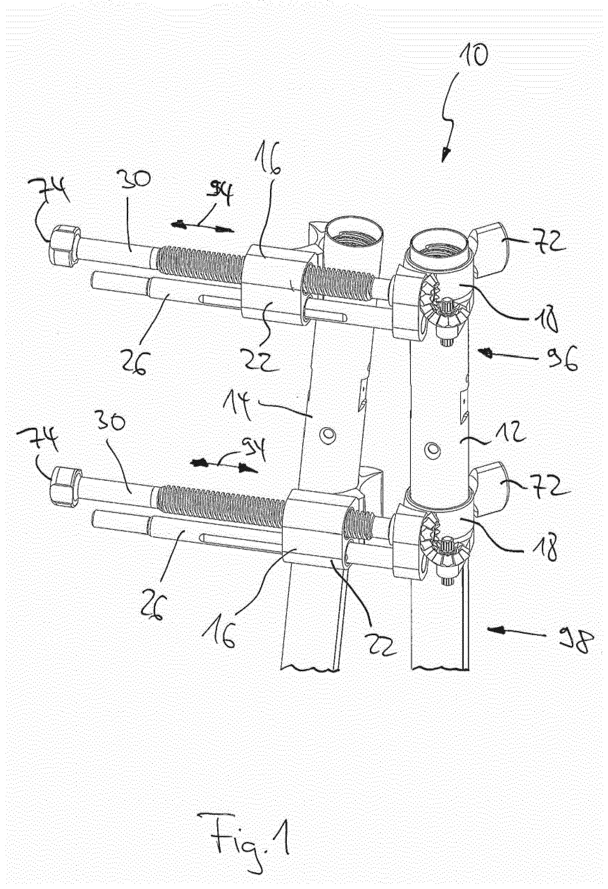

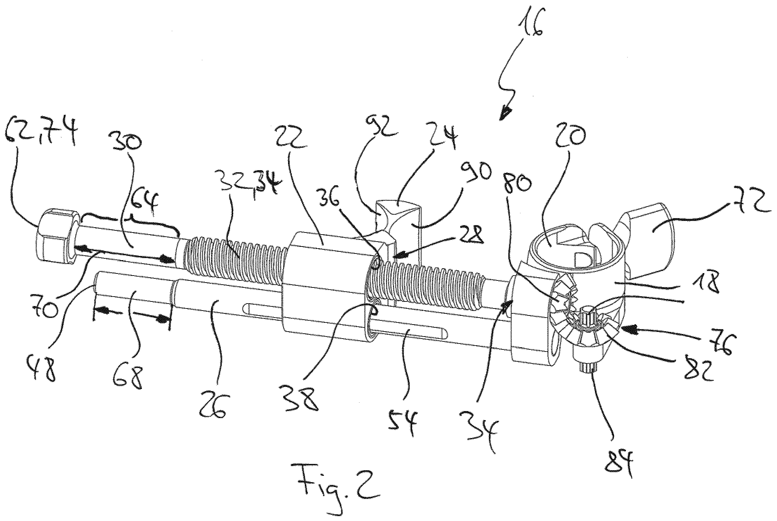

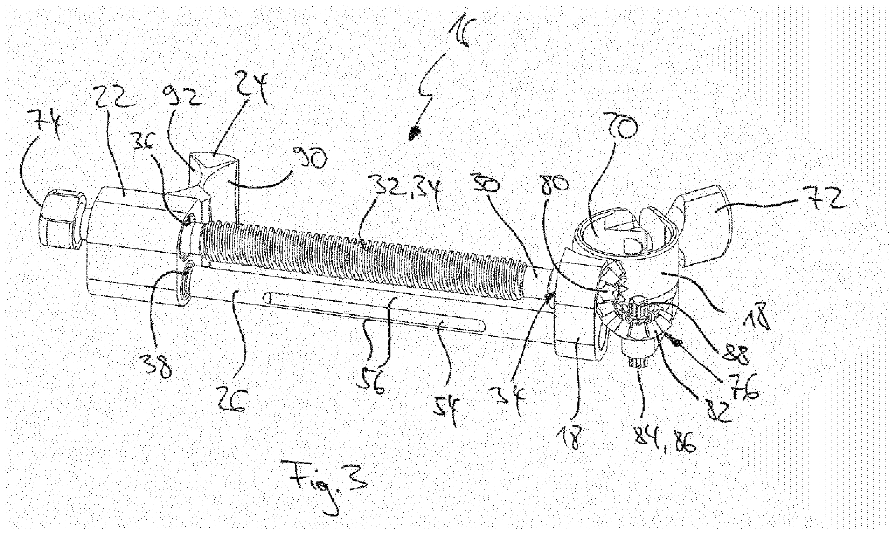

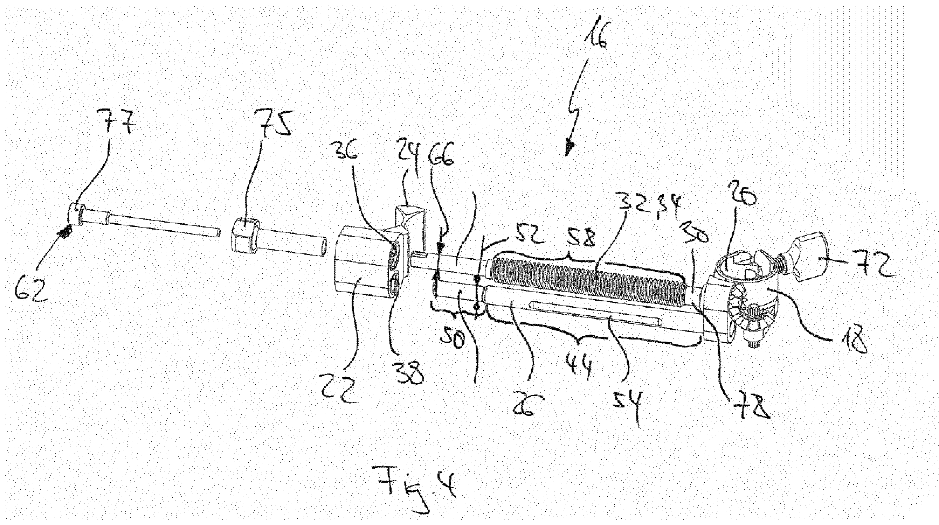

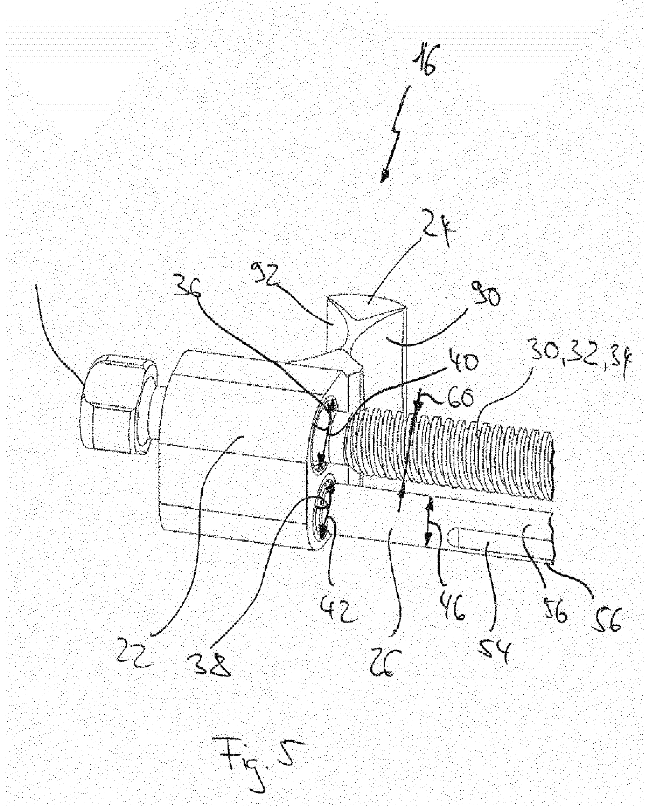

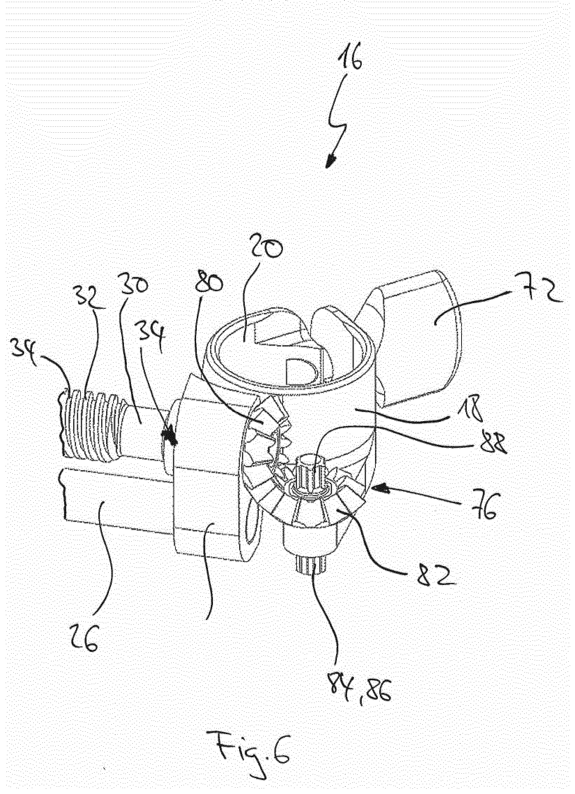

Figur 1 zeigt eine erfindungsgemäße Vorrichtung 10 zum Durchführen einer Distraktion und/oder einer Kompression von Wirbelkörpern bei einer minimalinvasiven Wirbelsäulenoperation. Die Vorrichtung 10 weist ein erstes und ein zweites zumindest abschnittsweise hülsenförmiges Verlängerungsteil 12, 14 mit einer jeweiligen Längsrichtung auf. Die hülsenförmigen Verlängerungsteile 12, 14 sind jeweils an einem in den Figuren nicht gezeigten Knochenanker zwar lösbar, jedoch drehfest festlegbar, wobei die Knochenanker jeweils in einen der zu behandelnden Wirbelkörper einzubringen oder bereits eingebracht sind. Darüber hinaus weist die Vorrichtung 10 zwei Spreiz- oder Klemmeinrichtungen 16 auf, wobei in den Figuren 2 bis 6 eine Spreiz- oder Klemmvorrichtung in Alleindarstellung gezeigt ist, wobei Figur 2 eine Schrägansicht der Spreiz- oder Klemmeinrichtung 16 zeigt, wobei Figur 3 eine Schrägansicht der Spreiz- oder Klemmeinrichtung 16 in einer Spüllage zeigt und wobei Figur 4 eine teilweise Explosionsdarstellung der Spreiz- oder Klemmeinrichtung 16 zeigt, wobei die Spreiz- oder Klemmeinrichtung 16 für die Wiederaufbereitung so zerlegt werden sollte, wie in Figur 4 dargestellt. In den Figuren 5 und 6 sind jeweils Detailansichten der Spreiz- oder Klemmeinrichtung 16 gezeigt.

Wie sich aus den Figuren 1 bis 6 ergibt, weist die Spreiz- oder Klemmeinrichtung 16 ein Grundteil 18 mit einer Aufnahme 20 zum lösbaren Festlegen der Spreiz- oder Klemmeinrichtung an dem ersten Verlängerungsteil 12 auf. Ferner weist die Spreiz- oder Klemmeinrichtung 16 ein Spreiz- oder Klemmteil 22 mit einem Anlageabschnitt 24 auf, der zur spreizenden und/oder klemmenden Anlage an dem zweiten Verlängerungsteil 14 ausgelegt ist.

Die Spreiz- oder Klemmeinrichtung 16 weist darüber hinaus eine Führung 26, entlang derer das Spreiz- oder Klemmteil 22 gegenüber dem Grundteil 18 verlagerbar ist. Zudem weist die Spreiz- oder Klemmeinrichtung 16 ein Schraubgetriebe 28 (vgl. Figur 2) mit einer Antriebswelle 30 auf, das zur relativen Verlagerung des Spreiz- oder Klemmteils 22 gegenüber dem Grundteil 18 ausgelegt ist.

Die Antriebswelle 30 ist drehbar mit dem Grundteil 18 verbunden und weist eine Gewindespindel 32 des Schraubgetriebes 28 auf. Ferner weist das Spreiz- oder Klemmteil 22 ein in den Figuren nicht gezeigtes Mutterngewinde des Schraubgetriebes 28 auf. Dabei korrespondieren das Gewinde 34 der Gewindespindel 32 bzw. die Gewindespindel 32 selbst derart mit dem Mutterngewinde des Spreiz- oder Klemmteils 22, dass eine Rotation der Antriebswelle 30 in eine axiale Verlagerung des Spreiz- oder Klemmteils 22 gegenüber dem Grundteil 18 entlang der Führung 26 umgesetzt wird.

Zur drehbaren Verbindung der Antriebswelle 30 mit dem Grundteil 18 ist im Grundteil 18 ein Lagerabschnitt 34 (vgl. Figur 6) vorgesehen, der zur drehbaren Lagerung der Antriebswelle 30 ausgelegt ist.

Die Führung 26 ist stabförmig und als von der Antriebswelle 30 verschiedenes Teil ausgebildet. Die Führung 26 ist drehfest, insbesondere einstückig, mit dem Grundteil 18 verbunden und die Antriebswelle 30 ist parallel zur Führung 26 angeordnet, so dass eine Rotation der Antriebswelle 30 in eine axiale Bewegung des Spreiz- oder Klemmteils 22 entlang der Führung 26 umgesetzt werden kann.

Im Spreiz- oder Klemmteil 22 sind eine erste Aufnahmebohrung 36 für die Antriebswelle 30 und eine zweite Aufnahmebohrung 38 für die Führung 26 vorgesehen, wobei das Mutterngewinde des Schraubgetriebes 28 in der ersten Aufnahmebohrung 36 angeordnet ist. Die erste Aufnahmebohrung 36 weist einen Bohrungsdurchmesser 40 auf, wobei die zweite Aufnahmebohrung 38 einen Bohrungsdurchmesser 42 aufweist (vgl. Figur 5).

Um eine Wiederaufbereitung der Spreiz- oder Klemmeinrichtung 16 und damit der Vorrichtung 10 zu erleichtern, weist die Führung 26 einen ersten Führungsabschnitt 44 mit einem ersten Führungsdurchmesser 46 auf (vgl. Figur 5). An ihrem freien Ende 48 weist die Führung 26 einen zweiten Führungsabschnitt 50 mit einem zweiten Führungsdurchmesser 52 auf (vgl. Figur 4), der kleiner ist als der erste Führungsdurchmesser 46. Dieser erste Führungsdurchmesser 46 ist nur geringfügig kleiner als der Bohrungsdurchmesser 42 der zweiten Aufnahmebohrung 38, so dass eine möglichst gute Führung des Spreiz- oder Klemmteils 22 auf der Führung 26 ermöglicht werden kann.

Die Führung 26 weist im Bereich des ersten Führungsabschnitts 44 drei orthogonal zueinander angeordnete Abflachungen 54 auf. Dabei sind die Abflachungen 54 derart angeordnet, dass der erste Führungsabschnitt 44 Bereich der Abflachungen 54 dennoch im Querschnitt kreisringabschnittsförmige Gleitabschnitte 56 aufweist, die am Umfang der Führung 26 zwischen den Abflachungen 54 angeordnet sind. Somit kann einerseits ein möglichst gutes Gleiten der Führung 26 in der zweiten Aufnahmebohrung 38 des Spreiz- oder Klemmteils 22 gewährleistet werden, wobei andererseits Verschmutzungen durch bspw. menschliches Knochen- oder Gewebematerial einfach ausgespült werden können. Darüber hinaus zeigt Figur 4, wie die Vorrichtung 10 zusätzlich demontiert werden kann, um die Wiederaufbereitung zu verbessern. Dazu kann über eine Schraube 77 ein hülsenartiges Haltemittel 75, insbesondere ein Griff, entfernt werden, wobei anschließend das Spreiz- oder Klemmteil 22 von der Antriebswelle 30 bzw. von der Gewindespindel 32 entfernt werden kann. Alle Bauteile können dann einzeln der Wiederaufbereitung zugeführt werden.

Um eine Wiederaufbereitung der Spreiz- oder Klemmeinrichtung 16 und damit der Vorrichtung 10, insbesondere im Bereich der Gewindespindel 32 weiter zu erleichtern, weist die Antriebswelle 30 einen ersten Antriebsabschnitt 58 auf (vgl. Figur 4), in dem die Gewindespindel 32 angeordnet ist, die einen Gewindespindeldurchmesser 60 aufweist. Ferner weist die Antriebswelle 30 im Bereich ihres dem Grundteil 18 abgewandten freien Endes 62 einen zweiten Antriebsabschnitt 64 mit einem Antriebswellendurchmesser 66 auf, der kleiner ist als der Gewindespindeldurchmesser 60. Der zweite Führungsabschnitt 50 und der zweite Antriebsabschnitt 64 weisen eine Länge 68, 70 auf, die derart ausgelegt ist, dass das Spreiz- oder Klemmteil 22 vollständig vom ersten Führungsabschnitt 44 bzw. vom ersten Antriebsabschnitt 58 heruntergeschoben werden kann, so dass Verschmutzungen aus der ersten und zweiten Aufnahmebohrung 36, 38 entfernt werden können, wenn das Spreiz- oder Klemmteil 22 auf den zweiten Führungsabschnitt 50 bzw. den zweiten Antriebsabschnitt 64 heruntergeschoben ist. Um das Spreiz- oder Klemmteil 22 zur Wiederaufbereitung der Spreiz- oder Klemmeinrichtung 16 nicht unnötig weit verschieben zu müssen, weisen sowohl der erste Führungsabschnitt 44 und der erste Antriebsabschnitt 58, als auch der zweite Führungsabschnitt 50 und der zweite Antriebsabschnitt 64 in etwa dieselbe Länge auf.

Wie insbesondere in Figur 6 deutlich zu erkennen ist, ist die Aufnahme 20 als Ringklammer ausgebildet und derart ausgelegt, dass das erste hülsenförmige Verlängerungsteil 12 in seiner Längsrichtung klemmend darin aufgenommen werden kann. An der Aufnahme 20 ist dazu eine in den Figuren nicht gezeigte Klemmschraube mit einer Flügelmutter 72 angeordnet, wobei durch Anziehen der Flügelmutter 72 eine Klemmkraft von der Aufnahme 20 auf das in der Aufnahme 20 angeordnete erste hülsenartige Verlängerungsteil 12 ausgeübt werden kann (vgl. Figur 1).

Zum Verlagern des Spreiz- oder Klemmteils 22 weist die Antriebswelle 30 an ihrem dem Grundteil 18 abgewandten freien Ende 62 einen ersten Drehmomentantriebsabschnitt 74 auf. Der erste Drehmomentantriebsabschnitt 74 ist koaxial zu einer Mittellängsachse der Antriebswelle 30 angeordnet und als Innensechskant bzw. als Werkzeugansatzstelle ausgebildet. Wie sich der Explosionsdarstellung in Figur 4 entnehmen lässt, ist die Antriebswelle 30 mehrteilig ausgebildet, wobei am freien Ende 62 ein hülsenartiges Haltemittel 75, insbesondere ein Griff, auf die Antriebswelle 30 aufgeschoben wird, welches mittels einer Schraube 77 mit der Antriebswelle 30 verschraubt ist.

An der Spreiz- oder Klemmeinrichtung 16 ist ferner ein Winkelgetriebe 76 vorgesehen, das zum Antreiben der Antriebswelle 30 am einem dem freien Ende 62 abgewandten zweiten Ende 78 ausgelegt ist. Das Winkelgetriebe 76 ist im vorliegenden Fall als Kegelgetriebe mit zwei Kegelrädern 80, 82 ausgebildet.

Das erste Kegelrad 80 ist dabei drehfest mit der Antriebswelle 30 verbunden. Das zweite Kegelrad 82 ist an einem Lagerabschnitt des Grundteils 18 orthogonal zum ersten Kegelrad 80 auf einer zweiten Welle 84 drehbar gelagert angeordnet. Die zweite Welle 84 weist an ihren jeweiligen Enden einen zweiten als Außentorx ausgebildeten Drehmomentantriebsabschnitt 86 und einen dritten, ebenfalls als Außentorx ausgebildeten Drehmomentantriebsabschnitt 88 auf.

Um das zweite hülsenförmige Verlängerungsteil 14 manipulieren zu können, weist der Anlageabschnitt 24 einen Klemmabschnitt 90 und einen Spreizabschnitt 92 auf. Der Klemmabschnitt 90 ist dem Grundteil 18 zugewandt, wobei der Spreizabschnitt dem Grundteil 18 abgewandt ist. Der Klemmabschnitt 90 und der Spreizabschnitt 92 sind zur vollflächigen Anlage an den hülsenförmigen Verlängerungsteilen 12, 14, insbesondere am zweiten hülsenförmigen Verlängerungsteil 14 (vgl. Figur 1) ausgelegt, wobei der Anlageabschnitt 24 bei Blick in Längsrichtung des zweiten hülsenförmigen Verlängerungsteils 14 insgesamt doppelkonkav ausgebildet ist. Dabei ist die Oberfläche des in Längsrichtung gesehen konkav ausgebildeten Spreiz- oder Klemmabschnitts 90, 92 in sich nochmals konvex gewölbt, so dass das am Spreiz- oder Klemmabschnitt 90, 92 zur Anlage kommende zylindrische hülsenförmige Verlängerungsteil 14 nicht plan am Spreiz- oder Klemmabschnitt 90, 92 zur Anlage kommt. Vielmehr erfolgt beim Verschränken/Neigen des hülsenförmigen Verlängerungsteils 14 eine Art Abgleiten auf der in sich konvex gewölbten Oberfläche des konkaven Spreiz- oder Klemmabschnitts 90, 92, so dass eine Art Sattelgelenk bereitgestellt werden kann.

Zum Manipulieren der hülsenförmigen Verlängerungsteile 12, 14 kann die Spreiz- oder Klemmeinrichtung 16 wie folgt bedient werden. Wenn in der Aufnahme 20 der beiden in Figur 1 gezeigten Spreiz- oder Klemmeinrichtungen 16 jeweils das erste hülsenförmige Verlängerungsteil 12 geklemmt aufgenommen ist, kann durch Antreiben des ersten Drehmomentantriebsabschnitts 74 die Antriebswelle 30 derart in Rotation versetzt werden, dass das Spreiz- oder Klemmteil 22 entlang der Führung 26 in Richtung des in

Figur 1 gezeigten Doppelpfeils 94 verlagert wird. Wenn ein Chirurg keinen Zugang zum ersten Drehmomentantriebsabschnitt 74 hat, kann die Antriebswelle 30 alternativ dazu über das Winkelgetriebe 76 durch Antreiben des zweiten oder dritten

Drehmomentantriebsabschnitts 86, 88 in Rotation versetzt werden, um das Spreiz- oder Klemmteil 22 zu verlagern.

Bei der in Figur 1 gezeigten, oberen Spreiz- oder Klemmeinrichtung 16 wird der Klemmabschnitt 90 des Anlageabschnitts 24 dazu genutzt, das zweite hülsenförmige Verlängerungsteil 14 in einem oberen Bereich 96 hin zum ersten hülsenförmigen Verlängerungsteil 12 zu ziehen, wobei bei der in Figur 1 gezeigten, unteren Spreiz- oder Klemmeinrichtung 16 der Spreizabschnitt 92 des Anlageabschnitts 24 genutzt wird, um das zweite hülsenförmige Verlängerungsteil 14 in einem unteren Bereich 98 vom zweiten hülsenförmigen Verlängerungsteil 12 weg zu drücken bzw. als fixes Auf- oder Widerlager zu fungieren, so dass insgesamt eine Distraktion von Wirbelkörpern durch in die Wirbelkörper eingebrachte, in den Figuren nicht gezeigte Knochenanker erreicht werden kann, die am unteren Bereich 98 der hülsenförmigen Verlängerungsteile 12, 14 festgelegt sind.

## Patentansprüche

1. Vorrichtung (10) zum Durchführen einer Distraktion und/oder einer Kompression von Wirbelkörpern bei einer, insbesondere minimalinvasiven, Wirbelsäulenoperation, insbesondere mit einem ersten und einem zweiten, insbesondere zumindest abschnittsweise hülsenförmigen, Verlängerungsteil (12, 14) mit einer jeweiligen Längsrichtung, welches jeweils an einem Knochenanker zwar lösbar jedoch starr und drehfest festlegbar ist, wobei die Knochenanker jeweils in einen der zu behandelnden Wirbelkörper einzubringen oder bereits eingebracht sind, wobei eine Spreiz- oder Klemmeinrichtung (16) vorgesehen ist, wobei die Spreiz- oder Klemmeinrichtung (16) ein Grundteil (18) mit einer Aufnahme (20) zum lösbaren Festlegen der Spreiz- oder Klemmeinrichtung (16) an dem ersten Verlängerungsteil (12) aufweist und wobei die Spreiz- oder Klemmeinrichtung (16) ein Spreiz- oder Klemmteil (22) mit einem Anlageabschnitt (24) aufweist, der zur spreizenden und/oder klemmenden Anlage an dem zweiten Verlängerungsteil (14) ausgelegt ist, wobei die Spreiz- oder Klemmeinrichtung (16) eine Führung (26) aufweist, entlang derer das Spreiz- oder Klemmteil (22) gegenüber dem Grundteil (18) verlagerbar ist, wobei die Spreiz- oder Klemmeinrichtung (16) ein Schraubgetriebe (28) mit einer Antriebswelle (30) aufweist, das zur relativen Verlagerung des Spreiz- oder Klemmteils (22) gegenüber dem Grundteil (18) ausgelegt ist, **dadurch gekennzeichnet, dass** die Antriebswelle (30) an ihrem dem Grundteil (18) abgewandten freien Ende (62) einen ersten Drehmomentantriebsabschnitt (74) aufweist und dass ein Winkelgetriebe (76) vorgesehen ist, das zum Antreiben der Antriebswelle (30) an einem dem freien Ende (62) abgewandten zweiten Ende (78) ausgelegt ist.

2. Vorrichtung (10) nach Anspruch 1, wobei die Antriebswelle (30) drehbar mit dem Grundteil (18) verbunden ist, wobei die Antriebswelle (30) eine Gewindespindel (32) des Schraubgetriebes (28) aufweist und wobei das Spreiz- oder Klemmteil (22) ein Mutterngewinde des Schraubgetriebes (28) aufweist.

3. Vorrichtung (10) nach Anspruch 1 oder 2, wobei ein zweiter Drehmomentantriebsabschnitt (86, 88) vorgesehen ist, der am Grundteil (18) angeordnet ist.

4. Vorrichtung (10) nach Anspruch 3, wobei der zweite Drehmomentantriebsabschnitt (86, 88) orthogonal zum ersten Drehmomentantriebabschnitt (74) angeordnet ist.

5. Vorrichtung (10) nach wenigstens einem der vorstehenden Ansprüche, wobei das Winkelgetriebe (76) als Kegelgetriebe oder Kronenradgetriebe ausgebildet ist.

6. Vorrichtung (10) nach wenigstens einem der vorherigen Ansprüche, wobei die Führung (26) drehfest mit dem Grundteil (18) verbunden ist.

7. Vorrichtung (10) nach wenigstens einem der vorherigen Ansprüche, wobei die Antriebswelle (30) parallel zur Führung (26) angeordnet ist.

8. Vorrichtung (10) nach wenigstens einem der vorherigen Ansprüche, wobei im Grundteil (18) eine erste Aufnahmebohrung (36) für die Antriebswelle (30) vorgesehen ist und wobei im Grundteil (18) eine zweite Aufnahmebohrung (38) für die Führung (26) vorgesehen ist.

9. Vorrichtung (10) nach wenigstens einem der vorherigen Ansprüche, wobei die Führung (26) einen ersten Führungsabschnitt (44) mit einem ersten Führungsdurchmesser (46) aufweist und wobei die Führung (26) an ihrem freien Ende(48) einen zweiten Führungsabschnitt (50) mit einem zweiten Führungsdurchmesser (52) aufweist, der kleiner ist als der erste Führungsdurchmesser (46).

10. Vorrichtung (10) nach Anspruch 9, wobei die Führung (26) im Bereich des ersten Führungsabschnitts (44) wenigstens eine, vorzugsweise drei Abflachungen (54) aufweist, die orthogonal zueinander angeordnet sind.

11. Vorrichtung (10) nach wenigstens einem der vorstehenden Ansprüche, wobei die Antriebswelle (30) einen ersten Antriebsabschnitt (58) aufweist, in dem die Gewindespindel (32) angeordnet ist, die einen Gewindespindeldurchmesser (60) aufweist, und wobei die Antriebswelle (30) im Bereich des freien Endes (62) einen zweiten Antriebsabschnitt (64) mit einem Antriebswellendurchmesser (66) aufweist, der kleiner ist als der Gewindespindeldurchmesser (60).

12. Vorrichtung (10) nach Anspruch 9 oder 10 und 11, wobei der erste Führungsabschnitt (44) und der erste Antriebsabschnitt (58) in etwa dieselbe Länge aufweisen und wobei der zweite Führungsabschnitt (50) und der zweite Antriebsabschnitt (64) in etwa dieselbe Länge aufweisen.

13. Vorrichtung (10) nach wenigstens einem der vorherigen Ansprüche, wobei im Grundteil (18) ein Lagerabschnitt (34) vorgesehen ist, der zur drehbaren Lagerung der Antriebswelle (30) ausgelegt ist.

14. Vorrichtung (10) nach wenigstens einem der vorherigen Ansprüche, wobei der Anlageabschnitt (24) einen Klemmabschnitt (90) aufweist, der dem Grundteil (18) zugewandt ist, und wobei der Anlageabschnitt (24) einen Spreizabschnitt (92) aufweist, der dem Grundteil (18) abgewandt ist.

## Claims

1. Device (10) for carrying out a distraction and/or a compression of vertebral bodies during an in particular minimally invasive spinal surgery; in particular with a first and a second extension part (12, 14), which in particular are sleeve-like at least in portions and have a respective length direction, and which can be secured releasably but rigidly and rotationally fixedly to a respective bone anchor, wherein the bone anchor is to be inserted or is already inserted in a vertebral body to be treated; wherein a spreading or clamping device (16) is provided; wherein the spreading or clamping device (16) has a base part (18) with a receiver (20) for releasably fixing the spreading or clamping device (16) to the first extension part (12), and wherein the spreading or clamping device (16) has a spreading or clamping part (22) with a contact portion (24) which is designed for spreading and/or clamping contact on the second extension part (14); wherein the spreading or clamping device (16) has a guide (26) along which the spreading or clamping part (22) is displaceable relative to the base part (18); wherein the spreading or clamping device (16) has a helical gear (28) with a drive shaft (30) which is configured for relative displacement of the spreading or clamping part (22) with respect to the base part (18); **characterised in that** at its free end (62) facing away from the base part (18), the drive shaft (30) has a first torque drive portion (74), and that at a second end (78) facing away from the free end (62), an angular gear (76) is provided which is configured for driving the drive shaft (30).

2. Device (10) according to claim 1, wherein the drive shaft (30) is rotatably connected to the base part (18), wherein the drive shaft (30) comprises a threaded spindle (32) of the helical gear (28), and wherein the spreading or clamping part (22) comprises a nut thread of the helical gear (28).

3. Device (10) according to claim 1 or 2, wherein a second torque drive portion (86, 88) is provided which is arranged on the base part (18).

4. Device (10) according to claim 3, wherein the second torque drive portion (86, 88) is arranged orthogonally to the first torque drive portion (74).

5. Device (10) according to at least one of the preceding claims, wherein the angular gear (76) is configured as a bevel gear or contrate gear.

6. Device (10) according to at least one of the preceding claims, wherein the guide (26) is connected rotationally fixedly to the base part (18).

7. Device (10) according to at least one of the preceding claims, wherein the drive shaft (30) is arranged parallel to the guide (26).

8. Device (10) according to at least one of the preceding claims, wherein a first receiver bore (36) for the drive shaft (30) is provided in the base part (18), and wherein a second receiver bore (38) for the guide (26) is provided in the base part (18).

9. Device (10) according to at least one of the preceding claims, wherein the guide (26) has a first guide portion (44) with a first guide diameter (46), and wherein at its free end (48), the guide (26) has a second guide portion (50) with a second guide diameter (52) which is smaller than the first guide diameter (46).

10. Device (10) according to claim 9, wherein in the region of the first guide portion (44), the guide (26) has at least one, preferably three flattenings (54) which are arranged orthogonally to one another.

11. Device (10) according to at least one of the preceding claims, wherein the drive shaft (30) has a first drive portion (58) in which the threaded spindle (32) having a threaded spindle diameter (60) is arranged, and wherein in the region of the free end (62), the drive shaft (30) has a second drive portion (64) with a drive shaft diameter (66) which is smaller than the threaded spindle diameter (60).

12. Device (10) according to claim 9 or 10 and 11, wherein the first guide portion (44) and the first drive portion (58) have approximately the same length, and wherein the second guide portion (50) and the second drive portion (64) have approximately the same length.

13. Device (10) according to at least one of the preceding claims, wherein a bearing portion (34), which is designed for rotatable mounting of the drive shaft (30), is provided in the base part (18).

14. Device (10) according to at least one of the preceding claims, wherein the contact portion (24) has a clamping portion (90) facing the base part (18), and wherein the contact portion (24) has a spreading portion (92) facing away from the base part (18).

## Revendications

1. Dispositif (10) de mise en œuvre d'une distraction et/ou d'une compression de corps vertébraux dans une intervention chirurgicale sur la colonne vertébrale, en particulier mini-invasive, en particulier comprenant une première et une deuxième partie d'allongement (12, 14) qui est en forme de douille, en particulier au moins par sections, et présente une direction longitudinale respective et qui peut être fixée respectivement sur un ancre à os, certes, de manière amovible, mais rigide et solidaire en rotation, dans lequel les ancres à os sont à implanter chacun ou sont déjà implantés chacun dans un des corps vertébraux à traiter, dans lequel un dispositif d'expansion ou de serrage (16) est prévu, dans lequel le dispositif d'expansion ou de serrage (16) comprend une partie de base (18) ayant un logement (20) pour fixer de manière amovible le dispositif d'expansion ou de serrage (16) à la première partie d'allongement (12) et dans lequel le dispositif d'expansion ou de serrage (16) comprend une partie d'expansion ou de serrage (22) ayant une section d'appui (24) qui est conçue pour un appui à expansion et/ou à serrage sur la deuxième partie d'allongement (14), dans lequel le dispositif d'expansion ou de serrage (16) comprend un guidage (26) le long duquel la partie d'expansion ou de serrage (22) peut être déplacée par rapport à la partie de base (18), dans lequel le dispositif d'expansion ou de serrage (16) présente un engrenage à vis sans fin (28) ayant un arbre d'entraînement (30) qui est conçu pour le déplacement relatif de la partie d'expansion ou de serrage (22) par rapport à la partie de base (18), **caractérisé par le fait que**, à son extrémité libre (62) montrant dans la direction opposée à la partie de base (18), l'arbre d'entraînement (30) présente une première section d'entraînement de couple (74), et qu'un engrenage angulaire (76) est prévu qui est conçu pour entraîner l'arbre d'entraînement (30) à une deuxième extrémité (78) montrant dans la direction opposée à l'extrémité libre (62) .

2. Dispositif (10) selon la revendication 1, dans lequel l'arbre d'entraînement (30) est relié en rotation à la partie de base (18), dans lequel l'arbre d'entraînement (30) comprend une broche filetée (32) de l'engrenage à vis sans fin (28) et dans lequel la partie d'expansion ou de serrage (22) présente un filetage d'écrou de l'engrenage à vis sans fin (28).

3. Dispositif (10) selon la revendication 1 ou 2, dans lequel il est prévu une deuxième section d'entraînement de couple (86, 88) qui est disposée sur la partie de base (18).

4. Dispositif (10) selon la revendication 3, dans lequel la deuxième section d'entraînement de couple (86, 88) est disposée de façon orthogonale à la première section d'entraînement de couple (74).

5. Dispositif (10) selon au moins l'une des revendications précédentes, dans lequel l'engrenage angulaire (76) est réalisé sous la forme d'un engrenage conique ou d'un engrenage à couronne dentée conique.

6. Dispositif (10) selon au moins l'une des revendications précédentes, dans lequel le guidage (26) est relié de manière solidaire en rotation à la partie de base (18).

7. Dispositif (10) selon au moins l'une des revendications précédentes, dans lequel l'arbre d'entraînement (30) est disposé de façon parallèle au guidage (26).

8. Dispositif (10) selon au moins l'une des revendications précédentes, dans lequel un premier alésage de réception (36) pour l'arbre d'entraînement (30) est prévu dans la partie de base (18) et dans lequel un deuxième alésage de réception (38) pour le guidage (26) est prévu dans la partie de base (18).

9. Dispositif (10) selon au moins l'une des revendications précédentes, dans lequel le guidage (26) présente une première section de guidage (44) ayant un premier diamètre de guidage (46) et dans lequel le guidage (26) présente, à son extrémité libre (48), une deuxième section de guidage (50) ayant un deuxième diamètre de guidage (52) qui est inférieur au premier diamètre de guidage (46).

10. Dispositif (10) selon la revendication 9, dans lequel le guidage (26) présente au niveau de la première section de guidage (44) au moins un méplat, de préférence trois méplats (54) qui sont disposés de façon orthogonale les uns aux autres.

11. Dispositif (10) selon au moins l'une des revendications précédentes, dans lequel l'arbre d'entraînement (30) présente une première section d'entraînement (58) dans laquelle est disposée la broche filetée (32) qui présente un diamètre de broche filetée (60), et dans lequel l'arbre d'entraînement (30) présente au niveau de l'extrémité libre (62) une deuxième section d'entraînement (64) ayant un diamètre d'arbre d'entraînement (66) qui est inférieur au diamètre de broche filetée (60).

12. Dispositif (10) selon la revendication 9 ou 10 et 11, dans lequel la première section de guidage (44) et la première section d'entraînement (58) présentent à peu près la même longueur et dans lequel la deuxième section de guidage (50) et la deuxième section d'entraînement (64) présentent à peu près la même longueur.

13. Dispositif (10) selon au moins l'une des revendications précédentes, dans lequel une section de support (34) est prévue dans la partie de base (18), qui est conçue pour supporter de manière rotative l'arbre d'entraînement (30).

14. Dispositif (10) selon au moins l'une des revendications précédentes, dans lequel la section d'appui (24) comprend une section de serrage (90) qui montre vers la partie de base (18), et dans lequel la section d'appui (24) comprend une section d'expansion (92) qui montre dans la direction opposée à la partie de base (18).
